Europäisches Patentamt

**European Patent Office**

Office européen des brevets

(11) Publication number: **0 032 514**
**B1**

(12) **EUROPEAN PATENT SPECIFICATION**

(45) Date of publication of patent specification: **21.11.84**

(51) Int. Cl.³: **A 61 B 10/00, G 01 N 29/04**

(21) Application number: **80901334.5**

(22) Date of filing: **21.07.80**

(86) International application number:
**PCT/JP80/00164**

(87) International publication number:
**WO 81/00198 05.02.81 Gazette 81/04**

(54) **Ultrasonic wave Tomographic Imaging System.**

(30) Priority: **25.07.79 JP 94532/79**

(43) Date of publication of application:
**29.07.81 Bulletin 81/30**

(45) Publication of the grant of the patent:
**21.11.84 Bulletin 84/47**

(84) Designated Contracting States:
**DE FR GB SE**

(56) References cited:
**DE-A-2 607 108**
**JP-A-47 041 317**
**JP-A-50 124 487**
**JP-A-52 071 887**
**JP-A-52 107 188**
**US-A-3 565 057**
**US-A-3 937 066**
**US-A-3 979 711**

**P.S. GREEN: "Acoustical Holography" vol. 5,
Plenom Press, pages 493-498 New York,
London**

(73) Proprietor: **FUJITSU LIMITED**
**1015, Kamikodanaka Nakahara-ku**
**Kawasaki-shi Kanagawa 211 (JP)**

(72) Inventor: **MIYAZAKI, Junji**
**5-4, Ibukino Midori-ku**
**Yokohama-shi Kanagawa, 227 (JP)**
Inventor: **MIWA, Hirohide**
**6-7-10, Miyazaki Takatsu-ku**
**Kawasaki-shi Kanagawa, 213 (JP)**
Inventor: **SHIMURA, Takaki**
**29-44, Tsurukawa, 4-chome**
**Machida-shi Tokyo, 194-01 (JP)**

(74) Representative: **Muir, Ian R. et al**
**HASELTINE LAKE & CO. Hazlitt House 28**
**Southampton Buildings Chancery Lane**
**London WC2A 1AT (GB)**

(56) References cited:
**L.W. KESSLER: "Acoustical Holography", vol. 7,
Plenom Press New York, London, pages 79-81**
**PROCEEDINGS OF IEEE, vol. 67, no. 4, April
1979 New York, US J.F. HAVLICE et al.:
"Medical Ultrasonic Imaging: An Overview of
Principles and Instrumentation" pages 620-641**

Courier Press, Leamington Spa, England.

## Description

This invention relates to an ultrasonic wave tomographic imaging system which radiates an ultrasonic wave into an object and reproduces the internal conditions of said object as an image from the reflected wave, the transmitted wave or the refracted or scattered wave received from the object.

An ultrasonic wave tomographic imaging system radiates an ultrasonic wave into an object of which the internal conditions are to be studied, receives the reflected wave, the transmitted wave or the scattered wave returning from the inside of the object and produces therefrom an internal acoustic image of the object.

As a system for observing internal conditions in an object, X-ray diagnostic systems are widely used. An ultrasonic wave tomographic imaging system, as compared with such an X-ray diagnostic system, is, especially in the case of a human body as the object, non-destructive of internal organs and is less dangerous. Moreover, the ultrasonic system has the merit that it is suited to the diagnosis of soft organs of the human body.

As an ultrasonic wave tomographic imaging system, a camera system utilizing an ultrasonic wave lens has already been proposed. This technique is disclosed both in United States Patent 3,979,711 and in United States Patent 3,937,066—"Ultrasonic Camera System and Method".

This ultrasonic camera system has the merit that a desired region can be examined on a real time basis and movements of the image can be observed, and also it is superior to some other ultrasonic wave tomographic imaging systems.

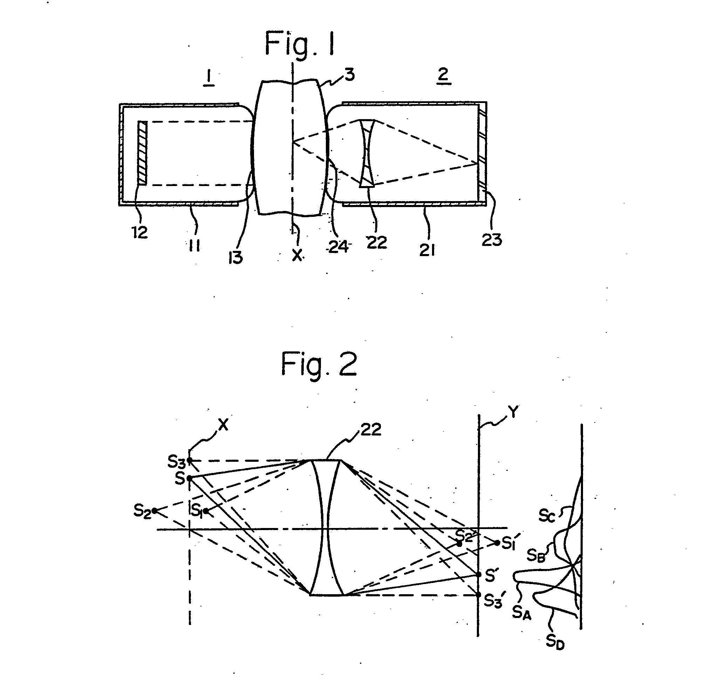

A prior ultrasonic wave tomographic imaging system using a camera is shown in Fig. 1 and is composed of ultrasonic wave generating means (a generator 1) and ultrasonic wave receiving means (a receiver 2) which are respectively provided on opposite sides of an object 3.

The generator 1 comprises an ultrasonic wave generator 12 consisting of an electric-acoustic transducer such as a crystal or PZT etc. housed in a case 11. A contact surface 13 with the object of the case 11 is composed of a flexible organic film having an acoustic impedance which is almost equal to that of the object.

The receiver 2 comprises an ultrasonic wave lens 22 which functions as the ultrasonic wave optical system and an acoustic transducer 23 housed in a case 21. A contact surface 24 with the object of the case 21 is also composed of an organic film as in the case of said contact surface 13.

The cases 11 and 21 are filled with a medium (for example water) which has an acoustic impedance almost equal to that of the object 3 (for example a human body).

The generator 1 and receiver 2 composed respectively as explained above are positioned in contact with the object 3 as indicated in Fig. 1, and the ultrasonic wave generator 12 radiates an ultrasonic wave into the object 3.

An ultrasonic wave acoustic image of object 3 is focussed on the acoustic transducer 23 by means of the ultrasonic wave lens 22.

The ultrasonic wave lens 22 has, as is well known, the function of converging the ultrasonic waves and focusses an acoustic image at the section X in the body at a position determined by the focal distance of the ultrasonic wave lens 22 and the distance between the lens 22 and the acoustic transducer 23.

An acoustic-visual image converter which utilizes an aluminum suspension liquid or an acoustic-electric transducer based on the piezoelectric transducer principle can be used as the acoustic transducer 23.

In the case of the ultrasonic wave tomographic imaging system of this type, an ultrasonic image of the imaging plane is correctly focussed on the transducer 23 but, in practice, ultrasonic wave images of the plane X are also reflected, refracted or scattered before reaching the surface of transducer 23. These images are superimposed and produce obscured images, i.e. the desired ultrasonic wave image is degraded because of noise, namely space noise wherein images of other planes than X are superimposed on the image of plane X and timing noise wherein additional images of plane X are superimposed on the direct image of plane X with some time delay because of repeated reflections, refractions and scatterings prior to reaching the transducer 23 from the generator 12.

Figure 2 explains the way in which such space noise images are superimposed. The ultrasonic waves reflected, refracted or scattered (hereinafter simply referred to as reflected) from the point S on the plane X form an image SA having an obscured space intensity distribution caused by reflection during travelling, and are focussed at the point S' on the surface Y of transducer 23.

Similarly, the ultrasonic waves reflected from the point $S_3$ on the plane X also form an image SD. at $S'_2$ having an obscured space intensity distribution. In addition, the ultrasonic waves reflected from the point $S_1$ which is nearer to the lens 22 than the plane X are focussed to the point $S'_1$ which is behind the surface Y, but form an imperfectly focussed image SB at the surface Y.

Additionally, ultrasonic waves reflected from the point $S_2$ which is further from the lens 22 than the plane X are focussed at the point $S'_2$ which is located in front of the surface Y and thereby form an imperfectly focussed image SC having a diverged space intensity distribution at the surface Y. As a result, it is a problem to be solved, in ultrasonic wave tomographic imaging systems of this type, that mutually focussed intensity distributions are superimposed as space noise on the desired image which is thereby obscured.

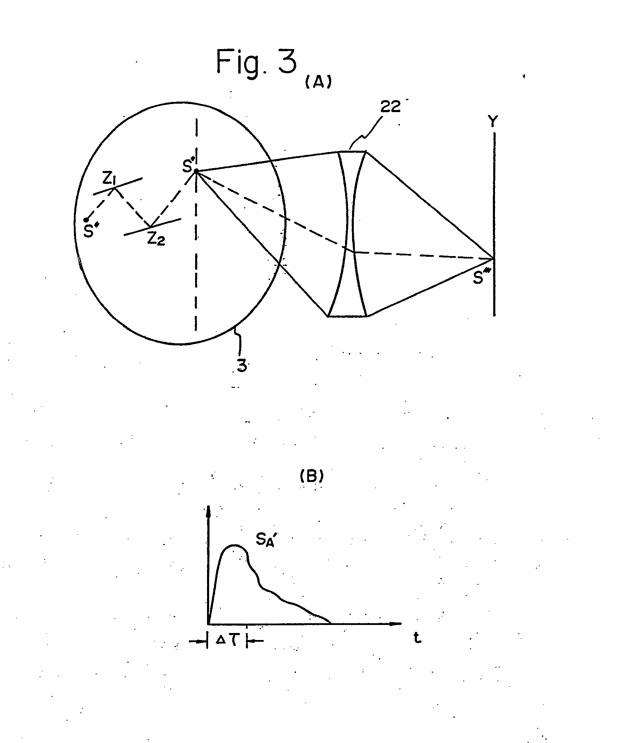

Figure 3 explains the way in which timing noise occurs. The ultrasonic waves reflected from the point S″ onto the plane X reach the point S‴ on the surface Y within a specified time ΔT. On the other hand, the ultrasonic waves reflected from the point S″ on the plane X are also multiply reflected at reflection surfaces $Z_1$, $Z_2$ and reach the point S‴ on the surface Y after a delay additional to ΔT via the point S″. It is another problem to be solved in an ultrasonic wave tomographic imaging system of this type that the time displaced images are as explained above superimposed as shown in Fig. 3(B), to form an intensity distribution image S′A along the time axis. Thus, the ultrasonic waves reflected from the point $S″_1$ comprise timing noise which results in an obscure image.

The systems disclosed in the prior mentioned U.S. patent specifications seek to reduce noise by gating the received signals.

The present invention seeks to provide an improved ultrasonic wave tomographic imaging system.

According to the present invention there is provided an ultrasonic wave tomographic imaging system incorporating ultrasonic wave scanning means for generating and scanning focussed ultrasonic waves across a tomographic plane of an object, ultrasonic wave receiving means for receiving ultrasonic wave signals from the tomographic plane, an ultrasonic lens arrangement for focussing an acoustic image from said tomographic plane onto the receiving means, and gate means for gating received signals of the receiving means to select those received signals that correspond to the scanning by said scanning means characterized in that the scanning means generates the ultrasonic waves in a direction lying in the tomographic plane.

In systems according to this invention, the image plane is regionally radiated by the ultrasonic wave.

With the availability of such regional radiation systems, the entire part may at first be diagnosed and thereafter the ultrasonic wave is regionally radiated only to the region to be examined carefully. The space noise of other non-radiated regions can, therefore, be avoided so as to make clearer the ultrasonic wave image obtained. For example, in the case of diagnosis of nephrolith, the ultrasonic wave is radiated at first to the entire part in order to obtain the total image and then radiated only to the region to be examined of the calculus. Thereby only a minute calculus becomes detectable by limiting the space noises reflected from the other regions such as ribs and diaphragm.

Moreover, this regional radiation is useful for combining an image of the entire part with less noise by selecting several regional images of the area to be examined. For this purpose, the tomographic plane is often scanned spot by spot with ultrasonic waves or scanned with a flat ultrasonic wave beam.

For a better understanding of the present invention reference will now be made, by way of example, to the accompanying drawings, in which:

Figure 1 illustrates diagrammatically the structure of a prior ultrasonic wave tomographic imaging system using a camera method;

Figure 2 is a diagram used to explain the occurrence of space noise in the prior ultrasonic wave tomographic imaging system of Fig. 1;

Figure 3 is a diagram used to explain the occurrence of timing noise in the prior ultrasonic wave tomographic imaging system of Fig. 1;

Figures 4 and 5 are diagrams used to explain the principle employed in this invention;

Figure 6 shows wave forms to explain a timing gate technique of the system according to the invention;

Figures 7 and 8 are diagram and graph respectively for explaining the gate time of the timing gate technique of Fig. 6;

Figures 9 and 10 are diagrams to explain the electronic focussing technique used in Fig. 4;

Figure 11 is a diagram to show an embodiment of the gain adjusting technique used in the structure indicated in Figure 4;

Figure 12 shows diagrams of another embodiment of the gain adjusting technique used in the structure indicated in Fig. 4;

Figure 13 is a block diagram of an embodiment of this invention;

Figure 14 is a diagram of a tomographic plane changing technique which may be used in this invention.

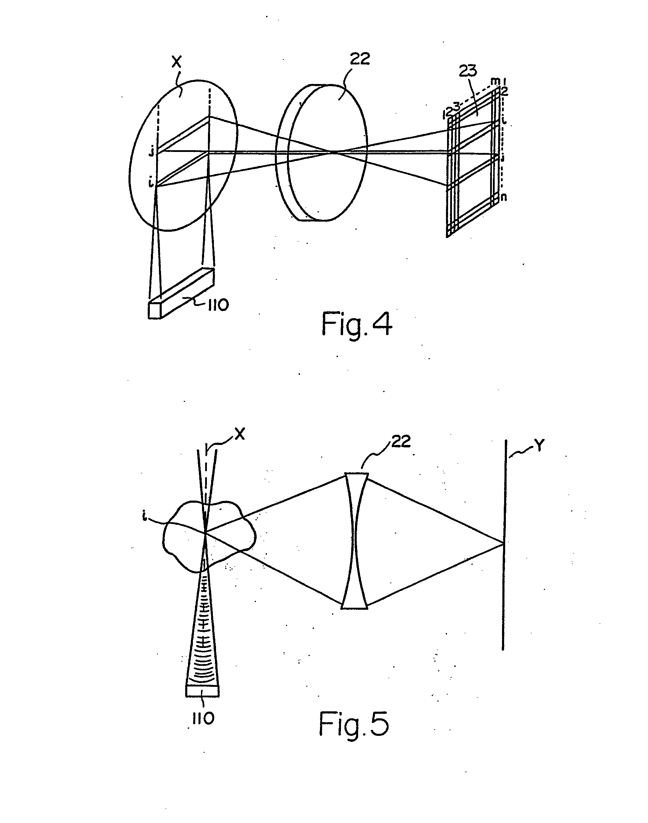

Figures 4 and 5 show the principle of the invention. In these Figures, 110 is a generator consisting of at least three rows of piezoelectric conversion elements in a matrix and it can generate an ultrasonic wave converging at a straight line i, for example, by operating with a phase difference between the drive signals for the central row of piezoelectric conversion elements and those for the two side rows. When this phase difference is varied, the straight line of the converged ultrasonic wave moves along the tomographic plane X. For instance, the straight line i may move to j. In other words, an electric focussing method is employed. When this electric focussing method is also employed along the row direction of the piezoelectric conversion element matrix, then spot-by-spot scanning becomes possible.

If a region having different acoustic impedance exists on the straight lines i or j, as the case may be, the focussed ultrasonic wave is reflected or scattered and an ultrasonic wave image is transmitted in a direction vertical to the tomographic plane X. The transmitted ultrasonic wave image is converged by the ultrasonic wave lens 22 and focussed on the transducer 23 of the image forming surface Y.

This transducer 23 is constituted, for example, by piezoelectric conversion element groups arranged in the form of an $m \times n$ matrix, and the ultrasonic wave pictures of the straight lines i and j in said plane X are respectively focussed on the piezoelectric conversion element groups of the i-th- and j-th rows of the transducer 23.

Therefore, a picture corresponding only to the irradiated region can be obtained by employing such an electric space gate method that when the ultrasonic wave is radiated to the straight line i, the output of piezoelectric conversion element groups in the i-th row is extracted at the time when such an ultrasonic wave image reached the transducer 23, and when the ultrasonic wave is radiated to the straight line j, the corresponding output of piezoelectric conversion element groups in the j-th row is extracted.

As an alternative, it is possible to employ a mechanical space gate method wherein a mask plate is used having a slit which makes it possible only to receive an image at the i-th row position when the ultrasonic wave is radiated to the straight line i and wherein the slit is moved in accordance with the sequential radiation of lines in the tomographic plane.

Similarly, a mechanical space gate means can be used, when an ultrasonic wave receiving means is itself moved in accordance with the sequential radiation of the tomographic plane, so long as the transducer 23 is a one dimensional ultrasonic wave receiving means. What is more, it is certainly possible to use a mechanical space gate means where the image-forming plane is mechanically moved in accordance with the rotating motion of a well-known acoustic lens system such as the acoustic prism which is disclosed in USP 3,913,061.

When the ultrasonic wave is radiated regionally only to the region to be examined, then reflection, scattering and refraction from areas other than the irradiated region are drastically decreased and a clear image having less noise may be obtained.

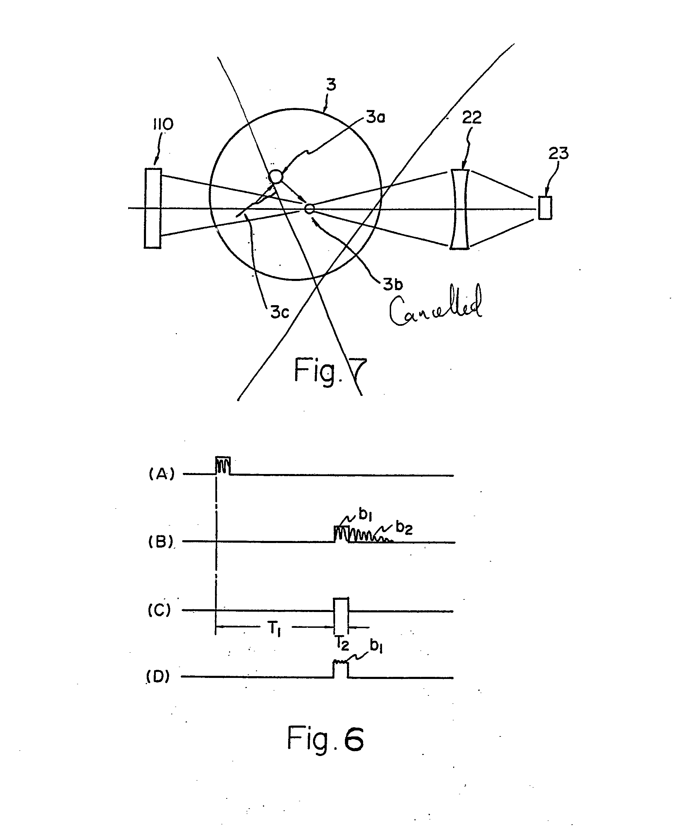

Figure 6 shows transmitted and received wave forms. In this Figure, (A) is a transmitted wave form generated by the generator 110, (B) is a received wave form of the transducer 23 before it is electrically gated, (C) is a gate signal wave form and (D) is a gated received wave form.

As indicated in Fig. 6(B), multiple reflected waves reach the regionally radiated area delayed with respect to the non-reflected radiated wave. In other words, an acoustic output b2 resulting from the multiple reflected waves is received delayed with respect to an acoustic output b1 resulting from the non-reflected ultrasonic wave.

Therefore, to obtain only the output b1 a gate signal (Fig. 6 (C)) may be used to eliminate the acoustic output b2.

The time $T_1$ from generation to gating can be obtained by dividing the transmission distance of the ultrasonic wave by the transmission speed.

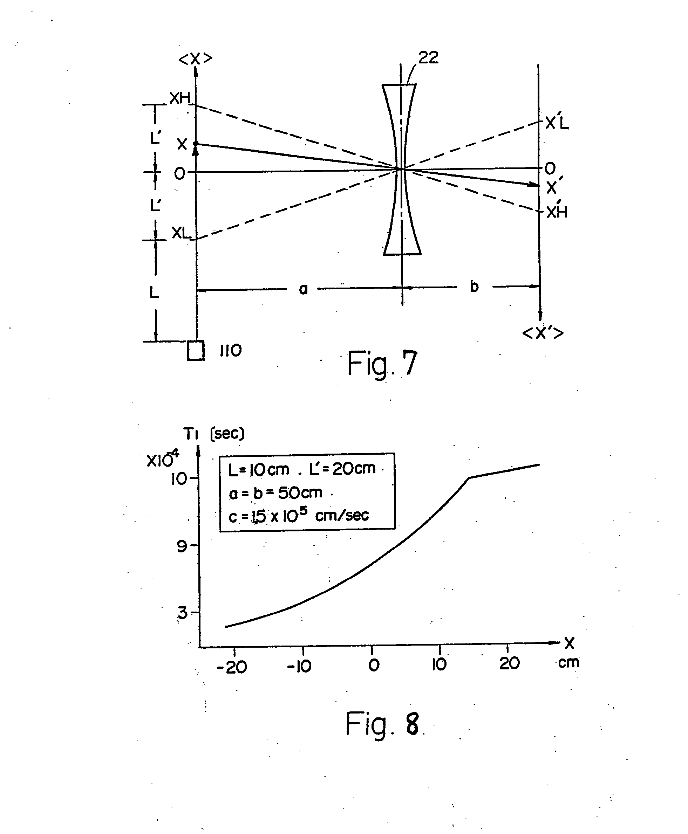

Figure 7 is for use in explaining a method of obtaining this time $T_1$ in the embodiment of the invention of Fig. 4. The ultrasonic wave generated from the generator 110 is transmitted in the direction $\langle X \rangle$ along the tomographic plane to be examined and is reflected at the position X, and then reaches the position X' on the axis $\langle X' \rangle$ where the transducer 23 is positioned having passed through the acoustic lens 22.

The symbols a, b, L and L' are respectively considered as the distance of each specified cross-section. When the transmission rate of ultrasonic wave is considered as C, the time $T_1$ is expressed by the following equation.

$$T_1 = [L + L' + x + (1 + b/a) \cdot \sqrt{a^2 + x^2}]/C$$

In Fig. 7, X'H and X'L respectively indicate the highest and lowest piezoelectric conversion elements of the matrix, and xH and xL indicate respectively the reflected points on the $\langle X \rangle$ axis of the ultrasonic waves received by the elements of X'H and X'L.

The ultrasonic wave radiation method employed is as shown in Fig. 4.

The graph of Fig. 8 shows the relation between $T_1$ and X for the case where the individual parameters are; $a = b = 50(cm)$, $c = 1.5 \times 10^5$ (cm/sec), $L = 10(cm)$, and $L' = 20(cm)$. As indicated in this graph, the time $T_1$ from generation of the ultrasonic wave to gating is determined by the position x of the focussed region in the plane X. In addition, the period of gating $T_2$ to be used is determined by the number of waves of the ultrasonic waves and the route difference of the acoustic lens.

An electric focussing method for electrically focussing the ultrasonic waves will now be explained in more detail.

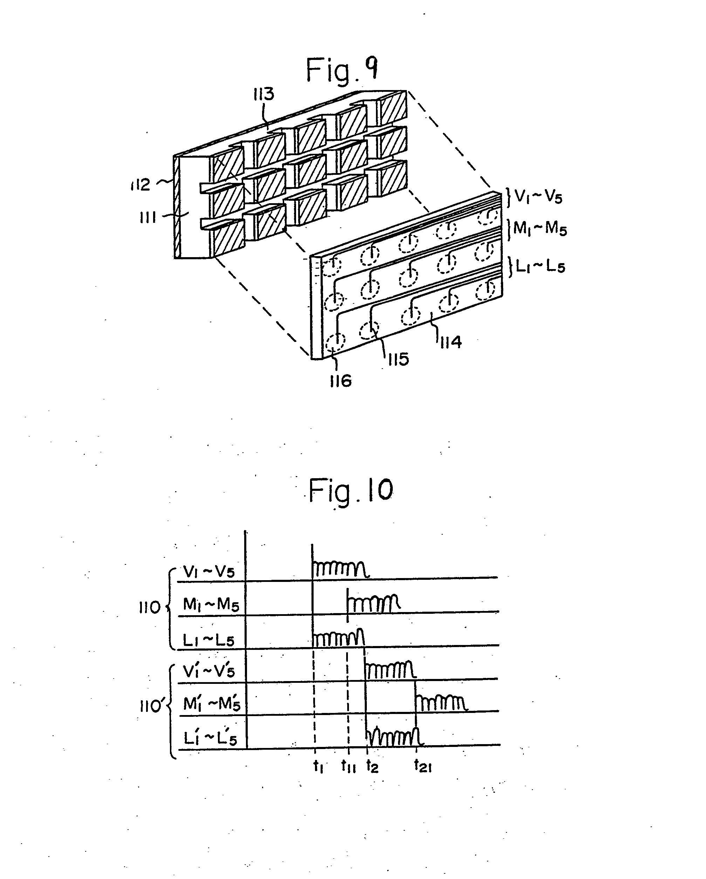

Figure 9 shows diagrammatically the detailed structure of the generator 110 and

Figure 10 shows the driving wave forms for the generator.

One side of a piezoelectric substrate 111 made of crystal or ceramic etc., is coated with a common electrode 112 and the other side is divided into 15 transducers (5×3), each of which has individual electrodes 113. On the bonding side of a wiring plate 114, which is bonded to individual electrodes, lead-out electrodes 116 are formed at positions corresponding to individual electrodes, whilst on the other side of the wiring plate 114, the connection wires $V_1$ to $V_5$, $M_1$ to $M_5$ and $L_1$ to $L_5$ are formed as printed wires. Each connection wire is connected to the corresponding lead-out electrode via holes 115 in the wiring plate 114.

When an in-phase drive signal is applied to the connecting wires $V_1$ to $V_5$ and $L_1$ to $L_5$, and a

delayed drive signal is applied to the wires $M_1$ to $M_5$ respectively, the ultrasonic wave is transmitted to the common electrode 112 in such a way that it converges towards the center from the outer two rows ($V_1$ to $V_5$ and $L_1$ to $L_5$). The desired line L at which the ultrasonic wave is focussed to a single line is determined by the phase delay given to the signals to the connecting wires $M_1$ to $M_5$. Therefore, the focussing line L can be sited at the desired position in the tomographic plane X by adjusting the amount of this phase delay. The focussed straight line L is moved over the desired range on the surface X and the reflected waves from the straight lines L positioned at equal interval are sampled and each wave is sequentially memorized as the data of a single raster scan of the display unit. Thereafter, when the stored data is read out and displayed in synchronization with the raster scanning of the display unit, display of the desired range on the plane X can be made with less noise.

With this form of ultrasonic wave scanning, the ultrasonic wave is attenuated and the received wave becomes weaker the further the focussing line is from the generator. To compensate for this, the receiving sensitivity should be changed automatically in accordance with the distance from the generator to the straight line L.

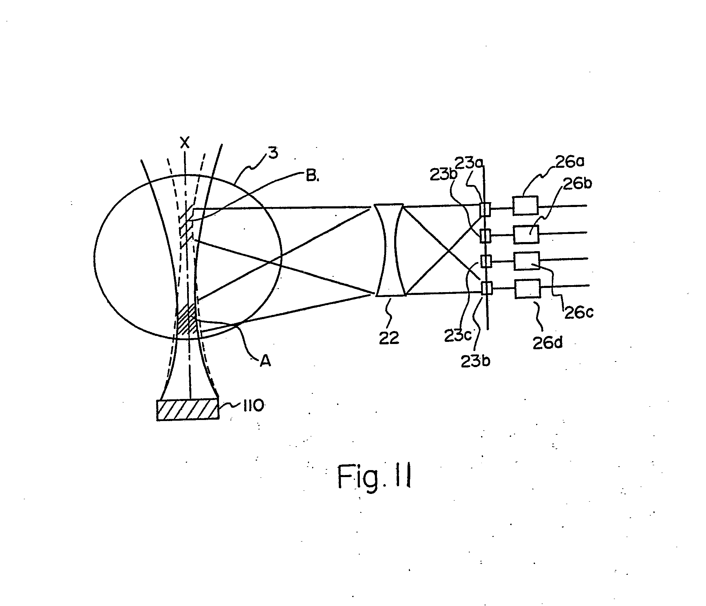

Figure 11 explains receiving sensitivity variation in an embodiment of this invention. In this figure, 23a to 23d are receiving elements shown with four rows (for simplification of explanation) as an example of a matrix receiving array. 26a to 26d are amplifiers corresponding to the receiving elements 23a to 23d. When the ultrasonic wave is focussed at point A of the focal plane X of the object 3, from the generator 110, its acoustic image is focussed on the receiving element 23a by means of the acoustic lens 22. As explained above, since the intensity of the ultrasonic wave is different at the focal points A and B, the difference must be corrected at the receiving side. In other words, the gain of the amplifier 26a is set in accordance with the distance $L_2$ between the focal point A and generator 110. The gain of the amplifiers 26b, 26c is similarly set in accordance with the distance between the corresponding focal point and the generator 110. In this embodiment, the amplifier 26d has maximum gain whilst the amplifier 26a has minimum gain. The gain of the amplifier for each row of the matrix array is in accordance with the above explanation.

Gain setting is not necessary if intensity differences are suppressed by using a non-linear circuit such as a log-amplifier.

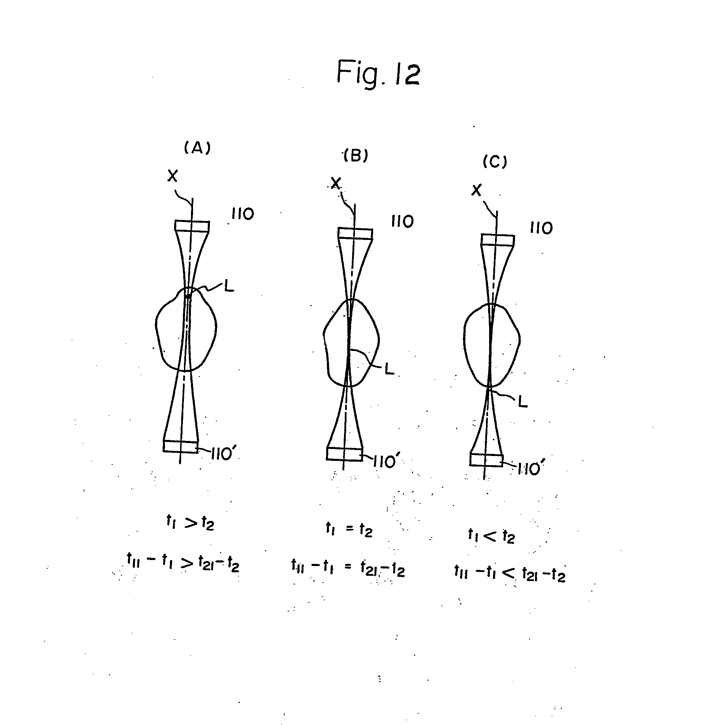

The intensity of the focussed ultrasonic waves becomes independent of the distance from the generators to the straight line L by providing two generators facing each other in the vicinity of the plane X. Figure 12 shows an example where two facing generators are used.

Two generators 110 and 110' like the one in Fig. 10 are provided face to face in the vicinity of the plane X.

The focussing line L moves as indicated in Fig. 12 (A) to (C) in accordance with the relation of the timings $t_1$, $t_{11}$, $t_2$ and $t_{21}$ as shown in Fig. 10 for the drive signal applied ho each row of vibrators of the pair of generators 110 and 110'. Since the pair of ultrasonic wave generators are located face to face vertically, the sum of the intensities of the ultrasonic waves from the generators, because of the symmetry, is substantially constant with a difference of intensity due to attenuation being substantially cancelled.

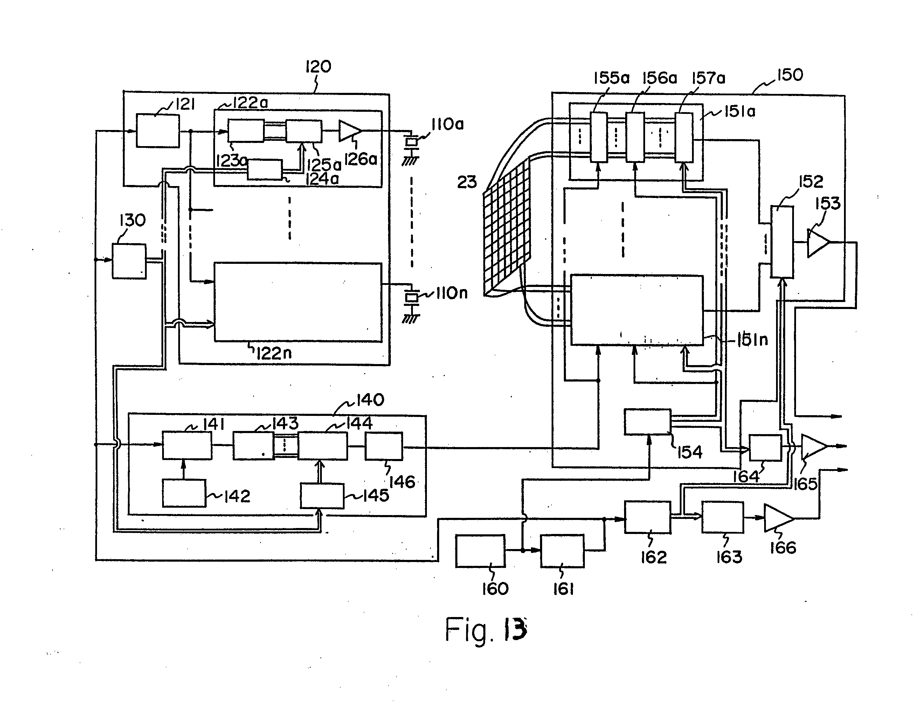

Figure 13 illustrates the block diagram of one embodiment of the invention.

In this figure, 120 is an electronic focussing circuit, this circuit driving piezoelectric conversion elements 110a to 110n of a generator 110, based on the principle of Fig. 9 with respect to electronic focussing and scanning. 130 is a scanning position address counter and 140 is a gate signal generator. 150 is an electronic gate circuit, which receives outputs of piezoelectronic conversion elements of each row and column of the receiving piezoelectric conversion element array 23 and samples the output of piezoelectric conversion elements in the specified row with the address signal and the output of gate signal generator 140. 160 is a reference clock oscillator, 161 a 256-bit counter for generating a horizontal synchronization signal, 162 a horizontal synchronization signal address counter, 163 and 164 digital-analog converters; and 165, 166 are amplifiers.

The electronic focussing circuit 120 is composed of an RF wave generator 121 and drive circuits 122a to 122n for individually driving the piezoelectric conversion element groups 110a to 110n of each row.

When an output of the horizontal synchronization signal counter 161 is input to the RF wave generator 121, it generates a burst wave consisting of a plurality of sine waves. Usually, two or three waves are used as the burst wave.

An output of the RF wave generator 121 is fed as input to each of the drive circuits 122a to 122n.

The drive circuits 122a to 122n comprise respectively delay circuits 123a to 123n, address memories 124a to 124n, multiplexer circuits 125a to 125n and amplifiers 126a to 126n.

The address memories 124a to 124n are controlled by a count value which is the output of the address counter 130, and the address counter 130 counts the horizontal synchronization signals.

For example, if the number of scanning lines for the display screen is selected as 256, the address counter 130 is a 256-bit counter. Namely, the address counter 130 generates the address from 0 to 255 and this address is the address information of the read-only-memory of the address memories 124a to 124n.

The address memories 124a to 124n store the selection signals of multiplexer circuits 125a to 125n in individual addresses.

Meanwhile, the burst output of RF wave generator 121 is input to the delay circuits 123a to 123n. These delay circuits input in parallel to the multiplexer circuits 125a to 125n and 256 kinds of delayed output having different delay times.

The multiplexer circuits 125a to 125n receive the outputs of the aforementioned address memories 124a to 124n and each outputs the specified delayed output among these delayed outputs.

These delayed outputs are amplified by the amplifiers 126a to 126n and fed to individual piezoelectric conversion elements 110a to 110n. Thus, these elements are driven to generate ultrasonic waves.

Each address of the address memories 124a to 124n must store the selection signal for selecting the delayed output in such a way that the ultrasonic wave is focussed on the area corresponding to the address specified by the address counter 130.

The electronic focussing operation is performed as explained above, and thereby the tomographic plane is sequentially scanned.

A gate signal is also generated in synchronization with the electronic focussing operation by means of the horizontal synchronization signal.

The gate signal generator 140 comprises the shift register 141, variable time clock generator 142, delay circuit 143, multiplexer 144, address memory 145, consisting of a read-only-memory, and the gate width set-up circuit 146, consisting of a one-shot multi-vibrator.

The horizontal synchronization signal is delayed by the shift register and the delay time can be controlled by the output of the variable time clock generator 142. The delay time of the shift register 141 is so adjusted that the minimum time $T_0$ of the ultrasonic wave generated from the generator 110 required for passing through a human body 3 to reach the receiving element 23 via the lens is obtained.

The horizontal synchronization signal delayed by the shift register 141 is fine-adjusted for its delay time by the delay circuit 143. This delay time compensates, in the case where the scanning line is selected as 256 for example, for a difference of the minimum times in said scanning operations for each focussed scanning.

For this reason, the delayed outputs of a number of corresponding to the scanning lines, for example 256, are output from the delay circuit 143.

These outputs are input to the multiplexer circuit 144. On the other hand, the address for focussing and scanning is given from the address counter 130. The address register 145 receives such address and outputs a selection signal to the multiplexer circuit 144 in order to select the delayed output which is most suitable for the scanning of such address.

The output of this multiplexer circuit 144, is lengthened, to the time required for the number of RF waves, at the gate width set-up circuit 146 and then output as the gate signal.

Meanwhile, the electronic gate circuit 150 is connected to each piezoelectric conversion element of the piezoelectric conversion matrix 23 of n-rows and m-columns. The outputs of the piezoelectric conversion elements of row 1, column M are input to the receiving circuit 151a and the outputs of the piezoelectric conversion elements of the n-th row and m-th column are sequentially input to the receiving circuit 151n.

The receiving circuits 151a to 151n are respectively composed of gate circuits 155a to 155n, memory circuits 156a to 156n, and multiplexer circuits 157a to 157n. The gate circuits 155a to 155n and memory circuits 156a to 156n are each respectively provided for m circuits. But they are indicated as single blocks in the figure.

The m gate units of each of the gate circuits 155a to 155n receive the data signal input.

The gate circuits 155a to 155n send m receiving inputs, as controlled by the gate signal, to the memory circuits 156a to 156n. The respective m memory units of the memory circuits 156a to 156n store these gated outputs.

Additionally, address counter 154 of the electronic gate circuit 150 receives clock signals from the reference clock generator 160, counts them and outputs the counted value. This address counter is designed as an m-bit counter, covering the number of columns of the piezoelectric conversion element matrix.

The output counted values are input to the multiplexers 157a to 157n and, therefore, the parallel outputs of the multiplexers 157a to 157n are converted to serial outputs. The memory circuits 156a to 156n are reset by a count-up signal of the address counter 154 for the next receiving input.

These n serial outputs are input in parallel to the multiplexer 152. Additionally, the multiplexer 152 receives a counted value of the address counter 162 which counts the horizontal synchronization signals.

Therefore, the multiplexer 152 outputs only one serial input corresponding to a counted value of the address counter 162 among n serial inputs. The address counter 162 is designed as an n-bit counter, covering the n-columns of the matrix. Therefore, when the number of scanning lines is selected as 256, n is also selected as 256.

An output of this multiplexer 152 is amplified by the amplifier 153 and is used by the display unit as the luminance signal.

Meanwhile, an output of the address counter 162 is converted to an analog signal by the digital-analog converter 163, amplified by the ampilifier 166 and used as the vertical (Y-axis) deflection signal for the display unit. Similarly, an output of the address counter 154 is also converted to an analog signal by the digital-analog converter 164, amplified by the amplifier 165 and used as the horizontal (X-axis) deflection signal for the display unit.

The above-mentioned operations are summarized below. The drive circuits 122a to 122n are controlled so that the ultrasonic waves are focussed and scanned to the scanning position of plane specified by the address counter 130, the gate signal which is delayed in accordance with the scanning position specified is generated by the gate signal generator 140, each receiving input of the m×n piezoelectric conversion element matrix 23 is sampled by this gate signal, the sampled signal is converted to a serial signal, and thereafter the serial signal corresponding to the row of the receiving position corresponding to said scanning position specified by the address counter 162 is output by the multiplexer circuit 152.

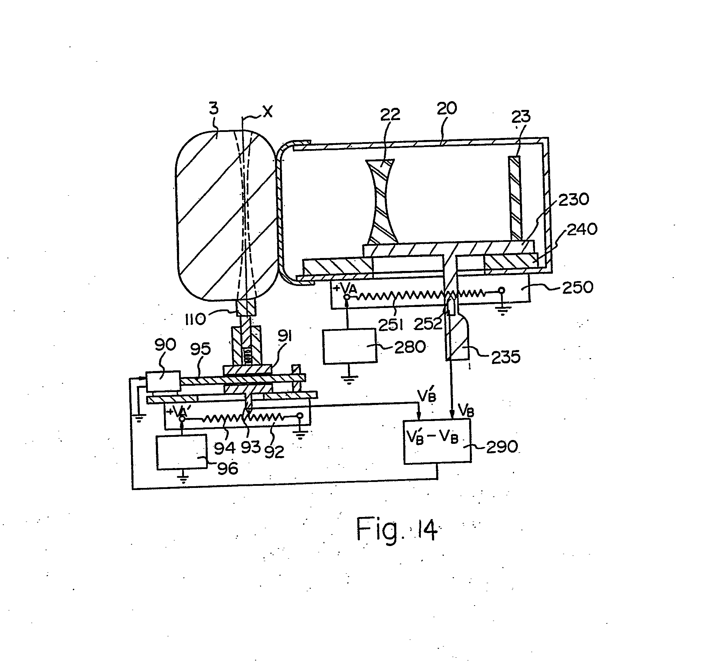

Figure 14 illustrates an embodiment of a tomographic plane changing technique which may be used in an arrangement in this invention.

The ultrasonic transducer 110 forming an ultrasonic wave generating means is provided in contact with an object 3 in the vicinity of the plane X to be examined of the object. A first means for moving the lens 22 and transducer 23 is provided by a movable base 230 which mounts the lens 22 and transducer 23 and which is manually moved by a handle 235, a sliding base 240 which is in contact with the movable base 230 and slides thereon, and a slidback 250 which detects the moving distance of the movable base 230. A second means for moving the ultrasonic wave transducer 110 is provided by a supporting base 91 which supports the ultrasonic wave transducer, a screw rod 95 which is engaged with a screwed hole in the supporting base 91, a DC motor 90 which rotates the screw rod 95 and the slidack 92 which detects the location of supporting base 91.

A DC voltage $+V_A$ is supplied to a slide resistor 251 in the slidback 250 from a voltage regulator 280, while a DC voltage $+V_{A'}$ is provided to a slide resistor 94 in the slidback 92 from a voltage regulator 96. A voltage $V_B$ detected by a sliding terminal 252 which operates in conjunction with the handle 235 and a voltage $V_{B'}$ detected by the sliding terminal 93 which operates in conjunction with the supporting base 91 are compared at a bridge circuit 290, and a voltage proportional to the difference $V_{B'}-V_B$ is supplied to the DC motor 90.

Here, it is supposed that two sliding resistors 251 and 94 have the same resistance value and are homogeneous, and that they are arranged in the same direction as indicated in the figure.

When the values of $V_A$ and $V_{A'}$ are set equal and the movable base 230 is set to a certain location, the location of supporting base 91 is determined so that $V_B$ and $V_{B'}$ become equal.

At this time, the second moving means as a whole is moved so that the ultrasonic wave transducer 1 is accurately located to the position of the plane X.

When the acoustic lens 22 and transducer 23 are moved together with the movable base 230 by means of the handle 135, the value of $V_B$ changes and the DC motor rotates until $V_{B'}$ becomes equal to $V_B$ and the ultrasonic transducer 110 moves together with the supporting base 91 for the same distance as mentioned above and in the same direction as the movable base 230. Additionally, the distance b between the lens 22 and transducer 23 is maintained to a constant value during such movement and, therefore, the location of the tomographic plane X also moves by the same distance and in the same direction as the movable base 230. Therefore, the ultrasonic transducer 110 is moved accurately to the location of the plane X.

In the above embodiment, the acoustic lens 22 and transducer 23 move together, but it is also possible for changing the location of the plane X to be examined to change the distance b betwen the acoustic lens 22 and the transducer 23 by moving the acoustic lens 22 or transducer 23. In this case, to enable the second moving means to respond, it is necessary that the moving distance of the lens or transducer is detected, the new value of b is calculated from the moving distance, and the moving distance x of the plane X is obtained by calculating a distance between the plane X and the acoustic lens from said value of b and the focal distance of the acoustic lens. The second means is then moved such that the ultrasonic transducer is moved by the distance x.

These detecting means, calculation means and moving means are all easily realized by well known methods.

**Claims**

1. An ultrasonic wave tomographic imaging system incorporating ultrasonic wave scanning means (110) for generating and scanning focussed ultrasonic waves across a tomographic plane (X) of an object, ultrasonic wave receiving means (23) for receiving ultrasonic wave signals from the tomographic plane, an ultrasonic lens arrangement (22) for focussing an acoustic image from said tomographic plane (X) onto the receiving means (23), and gate means (140, 150) for gating received signals of the receiving means (23) to select those received signals that correspond to the scanning by said scanning means (110) characterized in that the scanning means (110) generates the ultrasonic waves in a direction lying in the tomographic plane (X).

2. An ultrasonic wave tomographic imaging system according to claim 1 wherein said scanning means (110) focusses the generated ultrasonic waves onto a linear region (i, j) of said tomographic plane (X) and scans the plane (X) by moving the focussed line (i, j) along the plane.

3. An ultrasonic wave tomographic imaging system according to claim 1 or 2 wherein there are two scanning means (110, 110'), facing each other and generating ultrasonic waves towards each other along the tomographic plane (X), and both focussing their generated waves as substantially the same region along the plane (X).

**Revendications**

1. Système de formation d'images tomographiques par ondes ultrasonores, comprenant un dispositif de balayage d'ondes ultrasonores (110) pour produire et balayer des ondes ultrasonores focalisées sur un plan tomographique (X) d'un objet, un dispositif de réception d'ondes ultrasonores (23) destiné à recevoir des signaux d'ondes ultrasonores du plan tomographique, un dispositif de lentilles ultrasonores (22) pour focaliser une image acoustique provenant dudit plan tomographique (X) sur le dispositif de réception (23) et un dispositif d'aiguillage (140, 150) pour aiguiller des signaux reçus du dispositif de réception (23) afin de sélectionner les signaux reçus qui correspondent au balayage par ledit dispositif de balayage (110) produit les ondes ultrasonores dans une direction située dans le plan tomographique (X).

2. Système de formation d'images tomographiques par ondes ultrasonores selon la revendication 1, dans lequel ledit dispositif de balayage (110) focalise les ondes ultrasonores produites sur une région linéaire (i, j) dudit plan tomographique (X) et balaye le plan (X) en déplaçant la ligne focalisée (i, j) le long du plan.

3. Systeme de formation d'images tomographiques par ondes ultrasonores selon la revendication 1 Ou 2, dans lequel sont prévus deux dispositifs de balayage (110, 110') placés l'un en face de l'autre et produisant des ondes ultrasonores l'un vers l'autre le long du plan tomographique (X), et focalisant tous deux leurs ondes produites pratiquement dans la même région le long du plan (X).

**Patentansprüche**

1. Tomographie-System mit Abbildung durch Ultraschallwellen, mit einer Abtasteinrichtung (110) zur Erzeugung und Abtastung fokussierter Ultraschallwellen quer über eine tomographische Ebene (X) eines Objekts, Ultraschallwellenempfänger (23) zum Empfang von Ultraschallwellensignalen von der tomographischen Eben, einer Ultraschallinsenanordnung (22), zur Fokussierung eines akustischen Bildes von der genannten tomographischen Ebene (X) auf den Empfänger (23) und einer Torschaltung (140, 150) zum Austasten der von dem Empfänger (23) empfangenen Signale, um diejenigen empfangenen Signale auszuwählen, die der Abtastung durch die genannte Abtasteinrichtung (110) entsprechen, dadurch gekennzeichnet, daß die Abtasteinrichtung (110) die Ultraschallwellen in einer Richtung erzeugt, die in der tomographischen Eben (X) liegt.

2. Tomographie-System mit Abbildung durch Ultraschallwellen, nach Anspruch 1, bei dem die genannte Abtasteinrichtung (110) die erzeugten Ultraschallwellen auf einen linearen Bereich (i, j) der tomographischen Ebene (X) fokussiert und die Ebenen (X) durch Bewegen der fokussierten Linie (i, j) längs der Ebene abtastet.

3. Tomographie-System mit Abbildung durch Ultraschallwellen, nach Anspruch 1 oder 2, bei dem zwei Abtasteinrichtung (110, 110') vorgesehen sind, die einander gegenüber angeordnet sind und längs der tomographischen Ebene (X) Ultraschallwellen aufeinander zu erzeugen, und die beide ihre erzeugten Wellen auf im wesentlichen denselben Bereich längs der Ebene (X) fokussieren.

# Fig. 1

# Fig. 2

# Fig. 3 (A)

## (B)

Fig. 4

Fig. 5

Fig. 7

Fig. 6

Fig. 7

Fig. 8

## Fig. 9

## Fig. 10

Fig. II

# Fig. 12

(A)

$t_1 > t_2$

$t_{11} - t_1 > t_{21} - t_2$

(B)

$t_1 = t_2$

$t_{11} - t_1 = t_{21} - t_2$

(C)

$t_1 < t_2$

$t_{11} - t_1 < t_{21} - t_2$

Fig. 13

0032514

Fig. 14